# EUROPEAN PATENT SPECIFICATION

(11) **EP 3 043 244 B1**
(45) Date of publication and mention of the grant of the patent: **25.09.2019**
(21) Application number: 15150855.3
(22) Date of filing: 12.01.2015
(51) Int. Cl.: G06F 3/041, A61L 2/10

(54) **Device with a self-sterilizing touch screen**
Vorrichtung selbststerilisierendem Berührungsbildschirm
Dispositif avec un écran tactile autostérilisant

(43) Date of publication of application: 13.07.2016
(73) Proprietor: Ceyhan, Mesut, 16160 Osmangazi Bursa (TR); Vercan, Göktürk, 16960 Mudanya Bursa (TR); Dagli, Sevket Burcak, 16120 Nilüfer Bursa (TR); Emlek, Engin, 16140 Nilüfer Bursa (TR)
(72) Inventor: Ceyhan, Mesut, 16160 Osmangazi Bursa (TR); Vercan, Göktürk, 16960 Mudanya Bursa (TR); Dagli, Sevket Burcak, 16120 Nilüfer Bursa (TR); Emlek, Engin, 16140 Nilüfer Bursa (TR)
(74) Representative: Gislon, Gabriele

(56) References cited:
- WO-A1-2010/121031
- WO-A1-2014/142452
- US-A1- 2011 256 019
- US-A1- 2011 291 995

## Description

### Field of the invention

The present invention relates to a device provided with a touch screen such as smartphones, tablets, ATMs, kiosks, medical devices, industrial control panels, musical instruments, etc. The invention relates to the sterilization of a touchscreen and in particular the sterilization of the outer surface of the touch screen, i.e. the surface intended to be touched by an user.

### Background of the invention

Touch screens are employed in many devices, replacing mouse, keyboard and generic buttons. In fact by means of an electronic visual display the user can control a device through simple or multi-touch gestures by touching a screen.

Usually the electronic visual display is a flat screen, e.g. a LCD screen in which the outer screen, made generally in glass, is provided with a sensitive system for sensing the touch of an user. The sensitive system can be e.g. of the type resistive, capacitive, or of other types known in the art.

For example, WO2010121031 discloses optical touch screen wherein infrared rays emitted by IR-LEDs are used to detect the coordinates of a touchpoint on the screen.

A problem of the devices provided with touch screen is that the screen can accumulate a lot of bacteria or viruses due to several touches by one or more users. In mobile devices such as smartphones, touch screens are intended to be carried in proximity of the user face, for example to make a call. To overcome this problem are known devices to sterilize mobile devices, as mobile phones, to increase the cleanness of the device and promote the health of a user.

US2011256019 discloses a touchscreen comprising backlight UV-LEDs for sterilizing the touchscreen.

US2011291995 discloses a sterilizing device comprising a UV light source that emits UV rays such that the UV light rays are guided into a guiding member based on a total internal reflection.

KR20130074068A discloses a sterilization casing provided with a decontamination chamber in which a mobile phone can be placed. The decontamination chamber is provided with UV-LEDs and the mobile device is sterilized by means of UV rays emitted by the UV-LEDs. This method is not handy for the user because the sterilization chamber has big dimensions and the casing can be uncomfortable to be transported by the user.

To render the sterilizing system more compact, sterilization mobile phone covers are known. KR20140061594A discloses a flip cover for smartphones, wherein UV LEDs powered by the mobile phone battery are placed on the flip of the cover.

KR101132762B1 discloses a smartphone having a sterilizer provided with UV LEDs moveable towards the front side of a touch screen by rotation of a supporter.

These solutions can be uncomfortable for the user and can be unaesthetic for the mobile device as designed.

### Summary of the invention

Object of the present invention is to overcome the drawbacks of the prior art solutions above cited and to provide a device with a touchscreen provided with a sterilizing system able to sterilize the outer screen of the device without compromising the aesthetics of the device and without the need to cover the touch screen during the sterilization process.

A further object of the present invention is to provide a device with a touch screen wherein the sterilization of the outer screen can be scheduled by an user in a simple and intuitive manner.

These and other objects are reached by the present invention by means of a device according to claim 1 and the related dependent claims, and by a method for sterilizing a touch screen according to claim 11 and the related dependent claims.

In particular, according to the present invention, the device is provided with a touch screen comprising an outer screen for the touch by an user, a LCD screen, and a plurality of LEDs located below said outer screen for the backlighting of the LCD screen. The device is characterized in that at least one of said backlight LEDs is an UV-LED and in comprising guiding means to guide UV rays, emitted by said at least one UV-LED, towards the outer surface of said outer screen to sterilize the outer surface of the touch screen.

Guiding means comprise at least one reflector, located in one or more sides of the touch screen, the at least one reflector protruding from the outer surface of the touch screen in order to reflect the UV rays emitted by the at least one UV-LED towards the outer surface of the touch screen.

In fact, according to a further aspect of the present invention, the sterilization of the touch screen is carried out by means of UV rays emitted by at least one UV-LED placed internally to the device. In other words the outer surface of the touch screen is sterilized by means of UV rays emitted from behind said outer surface of the screen.

Typically the outer screen of a touch screen is made of glass. Glass is a material non-transparent to UV rays thus, UV rays emitted by one or more backlight UV-LEDs hitting the internal surface of a glass screen cannot go through said screen and do not reach its outer surface. According to an aspect of the present invention, UV rays can reach the outer screen of the device thank to guiding means comprising at least one portion of the outer screen made of a material transparent to UV rays (i.e. UV rays transparent material).

In one embodiment of the present invention, guiding means comprise the entire outer screen of the touch screen and the outer screen of the touch screen is entirely made of a material transparent to UV rays thus, the rays emitted by the backlight UV-LEDs can pass through the outer screen and reach the outer surface of the touch screen to sterilize the outer surface of the touch screen.

In another embodiment guiding means comprise one or more portions of the outer screen. Said one or more portions are made of a material transparent to UV rays and are located in one or more sides of said touch screen, preferably in each side. The remaining portions of screen are made of glass. In order to sterilize the entire outer surface of the outer screen, guiding means comprise at least one reflector, located in one or more sides of the touch screen, in correspondence of the portions transparent to UV rays (i.e. UV transparent portions) of the outer screen. UV rays, passing through the portions transparent to UV rays of the outer screen, are reflected by reflectors and are guided towards the outer surface of the screen thus, the entire outer surface of the touch screen is sterilized.

A first benefit of the present invention is that the backlight UV-LEDs can be controlled, e.g. by a processor of the device, in a simple manner. For example, in the case in which the device is a smartphone, backlight UV-LED can be controlled by means of a program (a so-called "app") with which the user can schedule the sterilization process. Furthermore smartphones are normally provided with proximity sensor to detect the vicinity of an user. Typically when the user is near to the screen (e.g. to make a call), the processor commands the shutdown of the backlights LED of the LCD screen to avoid that the touch of the user's face with the touch screen can send an undesired command (e.g. the shutdown of the call). At the same way the processor can command the shutdown of the backlight UV-LEDs to avoid that an user can be exposed to UV rays during a sterilization process.

The sterilizing system according to the present invention can be implemented in any type of device having a touchscreen with a LED backlighting as an LCD screen, thus not only mobile devices as tablet, PC and smartphones, but also ATMs, kiosks, or in general "fixed devices" (i.e. non-mobile devices). Starting from a present device, the sterilizing system of the present invention can be implemented in a simple manner, for example replacing one or more backlight LED with one or more UV-LED and changing the outer screen of the touch screen, for example with an outer screen made of quartz that is a material transparent to UV rays, and by providing guiding means to guide the UV light from the LED to the surface of the glass screen. Analogously, the processor of the device can command the shutdown of UV-LEDs, when backlight LEDs of the LCD screen are activated and/or in function of other sensors (e.g. accelerometers) present in the device for determining of the presence of an user using the device.

The sterilization system according to the present invention can be implemented without changing the original design (e.g. the shape of the casing) of the device.

According to a particular aspect of the present invention, UV-LEDs are preferably UV-C LEDs, i.e. that the UV rays emitted have a wavelength between 250 nm and 300 nm, preferably between 260 nm and 285 nm, more preferably between 260 nm and 270 nm.

UV-C rays will affect the ribosome of the pathogenic organisms, so they will not reproduce. After a period, pathogens will die. Irradiating the outer screen of the touch screen with UV-C rays, people can be protected from viruses, bacteria and pathogens. According to a further aspect of the present invention, backlight UV-LEDs can be activated by means of a button of the device (or a combination of buttons) and/or with a build-in application according to the decision of device producers.

### Brief description of the drawings

- Fig. 1 is a perspective view of an embodiment of the device according to the present invention;
- Fig. 2 is a perspective view of a second embodiment of the device according to the present invention;
- Fig. 3 is a perspective view in section of a third embodiment of the device according to the present invention;
- Fig. 4 is a block diagram of a system according to the invention.

### Best Modes for implementing the invention

For simplicity, in the following description reference will be made to a smartphone, but the present invention can be implemented on any device provided with a touch screen, wherein the screen is backlit by a plurality of backlight LEDs.

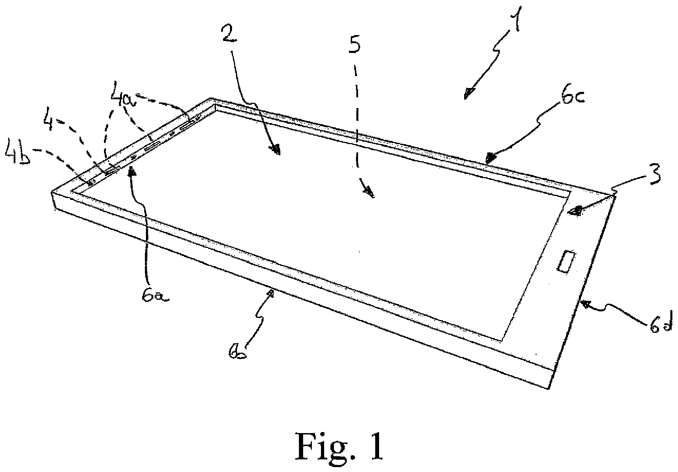

Figure 1 shows a device 1 (a smartphone) provided with a touch screen 2. Touch screen 2 comprises an outer screen 3 for the touch by an user, a LCD screen 5, and a plurality of LEDs 4 located below said outer screen 3 for the backlighting of said LCD screen 5.

Backlight LEDs 4b present in known touch screens emit white light for backlighting the LCD screen 5. The light emitted by backlight LEDs 4 passes through the LCD screen 5 and allow the displaying of an image (or in general an information) from the outer surface 3a of the outer screen 3 (generally made of glass, e.g. Gorilla Glass®).

Device 1 of the present invention comprises a touch screen 2 wherein at least one of said backlight LEDs 4 is an UV-LED 4a. In figure 1, the device 1 comprises seven backlight LEDs 4 of which three UV-LEDs 4a and four backlight LED 4b for emitting with light. The number and the location of the backlight LEDs 4a, 4b can vary in function of the touch screen 2 dimension and/or in function of the touch screen type. In figure 1, backlight LEDs 4 are located in a single side 6a of the touch screen 2, but further embodiment of the present invention can provide a touch screen 2 wherein backlight LEDs 5 can be located in each side 6a, 6b, 6c, 6d or in two opposite side 6a, 6d, in general in at least one side of the touch screen 2. Thus, the embodiment shown in figure 1 is only a possible configuration of the device 1 according the present invention. In general, the device 1 comprises at least one backlight UV-LED 4a.

In the case in which the touch screen 2 is realized with a technology wherein there is not the need of a backlighting (e.g. OLED technology), the device 1 according the present invention comprises a plurality of backlight LED 4 wherein all or at least one of said backlight LEDs 4 are UV-LEDs 4a.

The device 1 comprises guiding means 8 to guide UV rays 7, emitted by the UV-LEDs 4a, towards the outer surface 3a of the outer screen 3. Guiding means 8 comprise at least one portion 9 of the outer screen 3, said at least one portion 9 being made of a material transparent to UV rays.

In the embodiment shown in figure 1, the outer screen 3 is entirely made of a material transparent to UV rays thus, guiding means 8 comprise the entire outer screen 3 of the touch screen 2. Said material transparent to UV rays can be for example quartz. Further embodiments can provide a material transparent to UV rays different from quartz, as for example Sapphire, remaining in the scope of protection of the present invention.

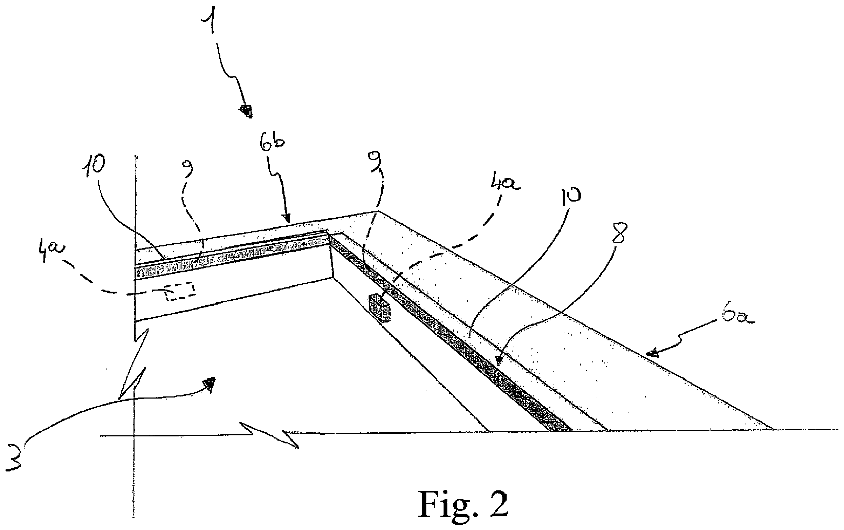

In figure 2 is shown a second embodiment of the device 1 according to the present invention wherein guiding means 8 comprise four portions 9 transparent to UV rays of the outer screen 3. Said four portions 9 are made of a material transparent to UV rays as quartz and are located in each side 6a-6d of the touch screen 2 as a sort of frame. The remaining portions of screen are made of glass. Further embodiments provide guiding means 8 comprising one or more portions 9 transparent to UV rays located in a single side 6a of the touch screen 2 or in two opposite sides 6a, 6c of the touch screen 2, in general, guiding means 8 comprise at least one portion 9 transparent to UV rays of outer screen 3 located in correspondence of the backlight UV-LEDs 4a.

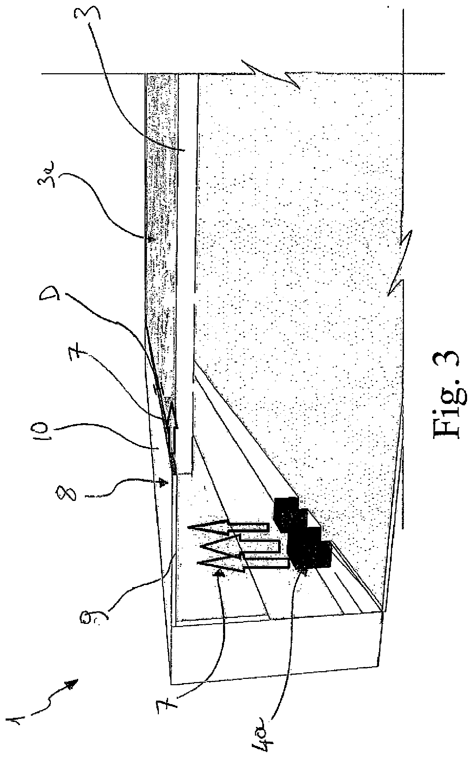

In the embodiment shown in figure 2, guiding means 8 comprise at least one reflector 10, located in one or more sides of the touch screen 2. In particular, reflectors 10 are located in correspondence of the portions 9 transparent to UV rays of the outer screen 3. As shown in figure 3, UV rays 7 emitted by UV-LEDs 4a pass through the portion 9 transparent to UV rays of the outer screen 3, are reflected by reflector and are guided towards the outer surface 3a of the outer screen 3 thus, the entire outer surface of the touch screen 2 can be irradiated by UV rays 7 and thus sterilized. In particular, reflectors 10 protrude from the outer surface of the touch screen in order to reflect the UV rays 7, emitted by UV-LEDs 4a, towards the outer surface 3a of the touch screen 2. In a preferred embodiment reflectors 10 cover the portion 9 transparent to UV rays of outer screen as a reflecting film in order to guide UV rays 7, passing through the transparent portion 9, to emerge towards the outer surface 3a of the touch screen.

Reflector 10 protrudes, from the outer surface 3a of the touch screen, by a distance D minor than 1 mm, preferably minor than 0,5 mm. In the embodiment shown in figure 3 reflectors 10 protrude by 0,2 mm, thus the design of device is not altered significantly.

UV-LEDs 4a are preferably UVC-LEDs, i.e. UV-LED emitting UV rays 7 having a wavelength between 250 nm and 300 nm. In a preferred embodiment UVC-LEDs 4a emit UV-C rays 7 with a wavelength of 265 nm. Said UVC-LEDs are SMD (surface mounting device) LED with a output power of about 0,5 mW at 20 mA forward current.

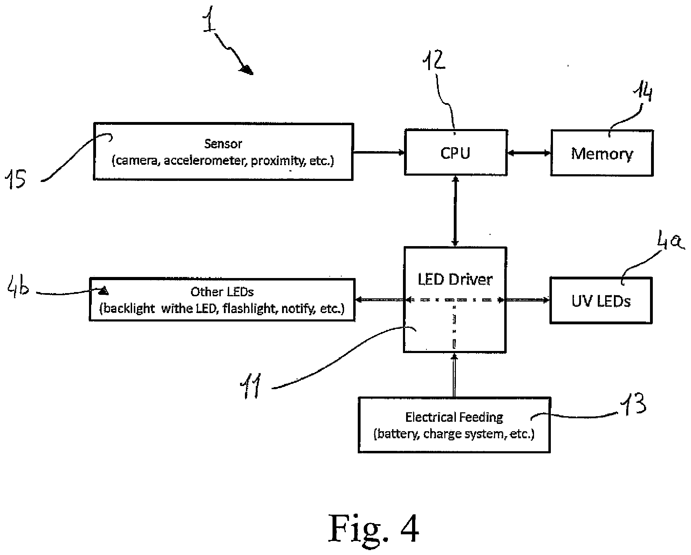

With reference to figure 4, the device 1 comprises a LED driver 11 to control the activation of said at least one UV-LED 4a independently from the other backlight LEDs 4b present in the device 1. Typically, a smartphone (or other devices provided with a touch screen) is provided with a LED driver 11 communicating with the processor 12 of the device 1. LED driver 11 is connected between a source of electrical feeding 13 (as a battery) and a plurality of LED. All kind of LEDs are connected to the LED driver 11, for example LEDs for backlighting of the LCD screen, flashlight LEDs if present on the device, LEDs for notify an event, etc.). By means of the LED driver 11 the processor 12 can command selectively the activation of said LEDs 4a, 4b. Analogously, the device 1 according to the invention comprise a LED driver 11 connected to backlight UV-LEDs 4a. The processor of the device can command, to the LED driver 11, the activating and/or deactivating of UV-LEDs 4a in function of the presence of an user who is using the device or who is in proximity of the touch screen.

The device 1 can comprise many sensors 15, e.g. a proximity sensor, a G sensor (an accelerometer or a gyroscope), a camera, in order to detect the presence of an user. The processor commands the shutdown of the backlight UV-LEDs when the presence of an user is detected to avoid that an user can be exposed to UV rays during a sterilization process. The processor has a set of safety rules (a program) in a memory 14 which the user can modifying, for example by means of an graphic interface realized with the touch screen. In one embodiment of the present invention, the user can schedule the sterilization process. The sterilization process will be carried out for at least one time period comprised between a starting instant and a final instant, wherein said starting instant and/or said final instant are settable by an user. In a further embodiment of the present invention the processor allows the activating of backlight UV-LEDs only if the device is on charge. Preferably, activation of UV-LEDs is permitted to be carried out when the charge level of the battery of the device (in this case a mobile device) is higher than a determined threshold.

Further embodiments can provide that backlight UV-LEDs can be activated by pressing a button of the device (or a combination of buttons) and/or with a build-in application according to the above cited safety rules.

## Claims

1. Device (1) provided with a touch screen (2) comprising an outer screen (3) for the touch by a user, a LCD screen (5), and a plurality of LEDs (4) located below said outer screen (3) for the backlighting of said LCD screen (5), wherein at least one of said backlight LEDs (4) is an UV-LED (4a), said device (1) comprising guiding means (8) to guide UV rays (7), emitted by said at least one UV-LED (4a), towards the outer surface (3a) of said outer screen (3) to sterilize said outer surface (3a) of said touch screen (2), **characterized in that** said guiding means (8) comprise at least one reflector (10), located in one or more sides (6a, 6b, 6c, 6d) of said touch screen (2), said at least one reflector (10) protruding from the outer surface (3a) of the touch screen (2) in order to reflect the UV rays (7) emitted by said at least one UV-LED (4a) towards the outer surface (3a) of the touch screen (2).

2. Device (1) according to claim 1, wherein said guiding means (8) comprise at least one portion (9) of the outer screen (3), said at least one portion (9) being made of a material transparent to UV rays.

3. Device (1) according to claim 2, wherein said at least one portion (9) transparent to UV rays of said outer screen (3) is located in one or more sides (6a, 6b, 6c, 6d) of said touch screen (2), preferably in each side.

4. Device (1) according to any one of the previous claims, wherein said outer screen (3) is made entirely of a material transparent to UV rays.

5. Device (1) according to any one of the previous claims, wherein said at least one reflector (10) is located in each side (6a, 6b, 6c, 6d) of said touch screen (2).

6. Device (1) according to any one of the previous claims wherein, said at least one reflector (10) protrudes, from the outer surface (3a) of said touch screen (2), by a distance (D) less than 1 mm, preferably less than 0,5 mm.

7. Device (1) according to any one
of the previous claims comprising a LED driver (11) to control the activation of said at least one UV-LED (4a) independently from the other backlight LEDs (4b) present in said device (1).

8. Device (1) according to any one of the claims from 2 to 7, wherein said material transparent to UV rays is quartz.

9. Device (1) according to any one of the previous claims, wherein said at least one UV-LED (4a) is a SMD UV-LED.

10. Device (1) according to any one of the previous claims, wherein said UV rays (7) emitted by said at least one UV-LED (4a) are UV-C rays (7), preferably with a wavelength between 250 nm and 300 nm, preferably between 260 nm and 285 nm, more preferably between 260 nm and 270 nm.

11. Method for sterilizing the outer surface (3a) of a touch screen (2) provided with an outer screen (3) for the touch by a user, comprising the step of:
a) emitting UV rays (7);
b) irradiate said outer surface (3a) of said touch screen (2) with said UV rays (7) emitted during said step a);
wherein said UV rays (7) are emitted from behind said outer surface (3a) of said outer screen (3), said method being **characterized in** comprising a step c) of guiding said UV rays (7), emitted during said step a), towards the outer surface (3a) of said touch screen (2) by said guiding means (8) which comprise at least one reflector (10) located in one or more sides (6a, 6b, 6c, 6d) of said touch screen (2), said at least one reflector (10) protruding from the outer surface (3a) of the touch screen (2) in order to reflect said UV rays (7) towards the outer surface (3a) of the touch screen (2).

12. Method according to claim 11, comprising a step d) of detecting the presence of a person in proximity of said touch screen (2), wherein said step a) of emitting said UV rays (7) is carried out when the presence of a person is not detected during said step d).

13. Method according to claim 11 or 12, wherein said step a) of emitting said UV rays (7), is carried out for at least one time period comprised between a starting instant and a final instant, wherein said starting instant and/or said final instant are settable by a user.

14. Method according to any one of the claims from 11 to 13, wherein said step a) is operated by pressing at least one button and/or by touching at least one portion of said touch screen (2).

15. Method according to any one of the claims from 11 to 14, wherein said touch screen (2) is mounted on a mobile device (1), said step a) of emitting said UV rays (7) being carried out when said mobile device (1) is on charge.

## Patentansprüche

1. Mit einem Berührungsbildschirm (2) versehene Vorrichtung (1), umfassend eine Außenscheibe (3) zum Berühren durch einen Benutzer, einen LCD-Bildschirm (5) und mehrere unterhalb der Außenscheibe (3) befindliche LEDs (4) zur Hintergrundbeleuchtung des LCD-Bildschirms (5), wobei mindestens eine der Hintergrundbeleuchtungs-LEDs (4) eine UV-LED (4a) ist, wobei die Vorrichtung (1) Führungsmittel (8) zum Führen der durch die mindestens eine UV-LED (4a) emittierten UV-Strahlen (7) in Richtung der Außenfläche (3a) der Außenscheibe (3) zum Sterilisieren der Außenfläche (3a) des Berührungsbildschirms (2) aufweist, **dadurch gekennzeichnet, dass** die Führungsmittel (8) mindestens einen, an einer oder mehreren Seiten (6a, 6b, 6c, 6d) des Berührungsbildschirms (2) angeordneten Reflektor (10) aufweisen, wobei der mindestens eine Reflektor (10) von der Außenfläche (3a) des Berührungsbildschirms (2) absteht, um die von der mindestens einen UV-LED (4a) zur Außenfläche (3a) des Berührungsbildschirms (2) hin emittierten UV-Strahlen (7) zu reflektieren.

2. Vorrichtung (1) gemäß Anspruch 1, wobei die Führungsmittel (8) mindestens einen Abschnitt (9) der Außenscheibe (3) umfassen, wobei der mindestens eine Abschnitt (9) aus einem für UV-Strahlen durchlässigen Material besteht.

3. Vorrichtung (1) gemäß Anspruch 2, wobei der mindestens eine für UV-Strahlen durchlässige Abschnitt (9) der Außenscheibe (3) an einer oder mehreren Seiten (6a, 6b, 6c, 6d) des Berührungsbildschirms (2) angeordnet ist, vorzugsweise in jeder Seite.

4. Vorrichtung (1) gemäß einem der vorhergehenden Ansprüche, wobei die Außenscheibe (3) vollständig aus einem für UV-Strahlen durchlässigen Material besteht.

5. Vorrichtung (1) gemäß einem der vorhergehenden Ansprüche, wobei der mindestens eine Reflektor (10) auf jeder Seite (6a, 6b, 6c, 6d) des Berührungsbildschirms (2) angeordnet ist.

6. Vorrichtung (1) gemäß einem der vorhergehenden Ansprüche, wobei der mindestens eine Reflektor (10) von der Außenfläche (3a) des Berührungsbildschirms (2) mit einem Abstand (D) von weniger als 1 mm vorsteht, vorzugsweise von weniger als 0,5 mm.

7. Vorrichtung (1) gemäß einem der vorhergehenden Ansprüche, umfassend einen LED-Treiber (11) zum Kontrollieren der Aktivierung der mindestens einen UV-LED (4a) unabhängig von den anderen, in der Vorrichtung (1) vorhandenen Hintergrundbeleuchtungs-LEDs (4b).

8. Vorrichtung (1) gemäß einem der Ansprüche 2 bis 7, wobei das für UV-Strahlen durchlässige Material Quarz ist.

9. Vorrichtung (1) gemäß einem der vorhergehenden Ansprüche, wobei die mindestens eine UV-LED (4a) eine SMD-UV-LED ist.

10. Vorrichtung (1) gemäß einem der vorhergehenden Ansprüche, wobei die von der mindestens einen UV-LED (4a) emittierten UV-Strahlen (7) UV-C-Strahlen (7) sind, bevorzugt mit einer Wellenlänge zwischen 250 nm und 300 nm, bevorzugt zwischen 260 nm und 285 nm, besonders bevorzugt zwischen 260 nm und 270 nm.

11. Verfahren zum Sterilisieren der Außenfläche (3a) eines Berührungsbildschirms (2), der mit einer Außenscheibe (3) zum Berühren durch einen Benutzer versehen ist, umfassend den Schritt:
a) Aussenden von UV-Strahlen (7);
b) Bestrahlen der Außenfläche (3a) des Berührungsbildschirms (2) mit den während des Schritts a) emittierten UV-Strahlen (7);
wobei die UV-Strahlen (7) hinter der Außenfläche (3a) der Außenscheibe (3) austreten, wobei das Verfahren **dadurch gekennzeichnet ist, dass** es umfasst einen Schritt
c) Führen der während des Schritts a) emittierten UV-Strahlen (7) zur Außenfläche (3a) des Berührungsbildschirms (2) über die Führungsmittel (8), welche mindestens einen in einer oder mehreren Seiten (6a, 6b, 6c, 6d) des Berührungsbildschirms (2) angeordneten Reflektor (10) umfassen, wobei der mindestens eine Reflektor (10) von der Außenfläche (3a) des Berührungsbildschirms (2) absteht, um die UV Strahlen (7) in Richtung der Außenfläche (3a) des Berührungsbildschirms (2) zu reflektieren.

12. Verfahren gemäß Anspruch 11, umfassend einen Schritt d) des Erfassens der Anwesenheit einer Person in der Nähe des Berührungsbildschirms (2), wobei der Schritt a) des Emittierens der UV-Strahlen (7) ausgeführt wird, wenn keine Anwesenheit einer Person während des Schritts d) erfasst wird.

13. Verfahren gemäß Anspruch 11 oder 12, wobei der Schritt a) des Emittierens der UV-Strahlen (7) für mindestens einen zwischen einem Startzeitpunkt und einem Endzeitpunkt liegenden Zeitraum ausgeführt wird, wobei der Startzeitpunkt und/oder der Endzeitpunkt von einem Benutzer festgelegt werden können.

14. Verfahren gemäß einem der Ansprüche 11 bis 13, wobei der Schritt a) durch Drücken mindestens einer Taste und/oder durch Berühren mindestens eines Teils des Berührungsbildschirms (2) ausgeführt wird.

15. Verfahren gemäß einem der Ansprüche 11 bis 14, wobei der Berührungsbildschirm (2) auf einem mobilen Gerät (1) angebracht ist, wobei der Schritt a) des Emittierens der UV-Strahlen (7) ausgeführt wird, wenn das mobile Gerät (1) aufgeladen wird.

## Revendications

1. Dispositif (1) pourvu d'un écran tactile (2) comprenant un écran externe (3) pour l'effleurement par un utilisateur, un écran LCD (5) et une pluralité de DEL (4) situées en dessous dudit écran externe (3) pour le rétroéclairage dudit écran LCD (5), dans lequel au moins l'une desdites DEL (4) de rétroéclairage est une DEL UV (4a), ledit dispositif (1) comprenant des moyens de guidage (8) pour guider les rayons UV (7), émis par ladite au moins une DEL UV (4a) vers la surface externe (3a) dudit écran externe (3) afin de stériliser ladite surface externe (3a) dudit écran tactile (2), **caractérisé en ce que** lesdits moyens de guidage (8) comprennent au moins un réflecteur (10) situé sur un ou plusieurs côtés (6a, 6b, 6c, 6d) dudit écran tactile (2), ledit au moins un réflecteur (10) faisant saillie de la surface externe (3a) de l'écran tactile (2) afin de réfléchir les rayons UV (7) émis par ladite au moins une DEL UV (4a) vers la surface externe (3a) de l'écran tactile (2).

2. Dispositif (1) selon la revendication 1, dans lequel lesdits moyens de guidage (8) comprennent au moins une partie (9) de l'écran externe (3), ladite au moins une partie (9) étant constituée d'un matériau transparent aux rayons UV.

3. Dispositif (1) selon la revendication 2, dans lequel ladite au moins une partie (9) transparente aux rayons UV dudit écran externe (3) est située sur un ou plusieurs côtés (6a, 6b, 6c, 6d) dudit écran tactile (2), de préférence sur chaque côté.

4. Dispositif (1) selon l'une quelconque des revendications précédentes, dans lequel ledit écran externe (3) est constitué intégralement d'un matériau transparent aux rayons UV.

5. Dispositif (1) selon l'une quelconque des revendications précédentes, dans lequel ledit au moins un réflecteur (10) est situé sur chaque côté (6a, 6b, 6c, 6d) dudit écran tactile (2).

6. Dispositif (1) selon l'une quelconque des revendications précédentes, dans lequel ledit au moins un réflecteur (10) fait saillie de la surface externe (3a) dudit écran tactile (2) d'une distance (D) inférieure à 1 mm, de préférence inférieure à 0,5 mm.

7. Dispositif (1) selon l'une quelconque des revendications précédentes, comprenant un pilote DEL (11) pour commander l'activation de ladite au moins une DEL UV (4a) indépendamment des autres DEL de rétroéclairage (4b) présentes dans ledit dispositif (1).

8. Dispositif (1) selon l'une quelconque des revendications 2 à 7, dans lequel ledit matériau transparent aux rayons UV est le quartz.

9. Dispositif (1) selon l'une quelconque des revendications précédentes, dans lequel ladite au moins une DEL UV (4a) est une DEL UV SMD.

10. Dispositif (1) selon l'une quelconque des revendications précédentes, dans lequel lesdits rayons UV (7) émis par ladite au moins une DEL UV (4a) sont des rayons UV-C (7), de préférence d'une longueur d'onde entre 250 nm et 300 nm, de préférence entre 260 nm et 285 nm, de manière plus préférée entre 260 nm et 270 nm.

11. Procédé de stérilisation de la surface externe (3a) d'un écran tactile (2) pourvu d'un écran externe (3) pour l'effleurement par un utilisateur, comprenant les étapes consistant à :
a) émettre des rayons UV (7) ;
b) irradier ladite surface externe (3a) dudit écran tactile (2) avec lesdits rayons UV (7) émis au cours de ladite étape a) ;
dans lequel lesdits rayons UV (7) sont émis de derrière ladite surface externe (3a) dudit écran externe (3), ledit procédé étant **caractérisé en ce qu'**il comprend une étape c) de guidage desdits rayons UV (7) émis au cours de l'étape a) vers la surface externe (3a) dudit écran tactile (2) par lesdits moyens de guidage (8) qui comprennent au moins un réflecteur (10) situé sur un ou plusieurs côtés (6a, 6b, 6c, 6d) dudit écran tactile (2), ledit au moins un réflecteur (10) faisant saillie de la surface externe (3a) de l'écran tactile (2) afin de réfléchir lesdits rayons UV (7) vers la surface externe (3a) de l'écran tactile (2).

12. Procédé selon la revendication 11, comprenant une étape d) de détection de la présence d'une personne à proximité dudit écran tactile (2), dans lequel ladite étape a) d'émission desdites rayons UV (7) est effectuée lorsque la présence d'une personne n'est pas détectée au cours de ladite étape d).

13. Procédé selon la revendication 11 ou 12, dans lequel ladite étape a) d'émission desdits rayons UV (7) est effectuée pendant au moins une période de temps comprise entre un instant de départ et un instant final, dans lequel ledit instant de départ et/ou ledit instant final est ou sont réglables par un utilisateur.

14. Procédé selon l'une quelconque des revendications 11 à 13, dans lequel ladite étape a) est effectuée en pressant au moins un bouton et/ou en effleurant au moins une partie dudit écran tactile (2).

15. Procédé selon l'une quelconque des revendications 11 à 14, dans lequel ledit écran tactile (2) est monté sur un dispositif mobile (1), ladite étape a) d'émission desdits rayons UV (7) étant effectuée lorsque ledit dispositif mobile (1) est en charge.
